# EUROPEAN PATENT APPLICATION

(11) **EP 4 353 762 A1**
(43) Date of publication of application: **17.04.2024**
(21) Application number: 22820030.9
(22) Date of filing: 23.05.2022
(51) Int. Cl.: C08F 293/00, A61K 9/14, A61K 31/19, A61K 47/58, A61K 47/60, A61P 25/06, A61P 25/08, A61P 25/18, A61P 35/00, A61P 43/00

(54) **POLYMERIZED VALPROIC ACID AND USE THEREOF**

(30) Priority: 07.06.2021 JP 2021095296
(71) Applicant: University of Tsukuba, Ibaraki 305-8577 (JP)
(72) Inventor: NAGASAKI, Yukio, Tsukuba-shi, Ibaraki 305-8577 (JP); IKEDA, Yutaka, Tsukuba-shi, Ibaraki 305-8577 (JP); TAJIKA, Yuya, Tsukuba-shi, Ibaraki 305-8577 (JP)
(74) Representative: Kraus & Lederer PartGmbB
(86) International application number: PCT/JP2022/021073
(87) International publication number: WO 2022/259848

(57) **Abstract**

Provided is a valproic acid derivative for improving disadvantages or defects accompanying when valproic acid itself or the like is used as a medicine. Provided is a block copolymer which is a polymerized product of valproic acid and comprises a poly(ethylene glycol) segment and a poly(vinyl valproate) segment.

## Description

### Technical Field

The present invention relates to a polymerized valproic acid and use thereof, and more particularly relates to a poly(ethylene glycol)-*b*-poly(vinyl valproate) copolymer and use thereof in the medical field.

### Background Art

A sodium salt of valproic acid (or 2-propylpentanoic acid) (hereinafter sometimes abbreviated as VPA) is widely used as an antiepileptic drug, a therapeutic drug for the manic state of bipolar disorders, or a drug for suppressing the onset of migraine attack at present. In addition, since VPA inhibits abnormal activation of histone deacetylase (HDAC) and has an antitumor effect, several clinical trials have also been conducted. Furthermore, it has been reported that VPA is also effective against cancer cachexia (see Am J Physiol Cell Physiol 311: C101-C115, 2016).

VPA sodium is used as a first choice drug of antiepileptic drugs, yet has issues to be solved: for example, it is necessary to keep the blood concentration thereof constant; it must be taken one or more times a day without forgetting; overdose for the purpose of self-mutilation or the like; and the presence of side effects such as hepatopathy and teratogenicity. In order to solve such issues, there have been provided preparations, as clinically used controlled-release agents of sodium valproate, which contain a carrier, an additive or the like commonly used in the art, such as Mg metasilicate aluminate, hydroxypropylcellulose, Mg stearate, ethylcellulose, hypromellose, glycerin fatty acid ester, talc, white sugar, precipitated Ca carbonate, polyoxyethylene (105) polyoxypropylene (5) glycol, powdered acacia, or titanium oxide. In addition, as a modification of the active substance itself, valproic acid has not been specifically disclosed as an active ingredient, but a product in an orally administered form has been proposed in which an acidic active ingredient having a free carboxyl group and a polymer having a tertiary amino group are bonded to each other (see JP H09-511488 T).

### Summary of Invention

### Technical Problem

Although specific film-coating agents, controlled-release preparations containing various carriers or additives as described above, and the like have been proposed in order to solve the above-mentioned problems even slightly, it would be desirable to provide a means which can take further various forms.

### Solution to Problem

As a result of reviews on chemical derivatization of VPA in order to solve such problems, the present inventor has found that a block copolymer comprising a segment of a polymer wherein VPA is covalently bonded to a polymer main chain via an ester bond (sometimes abbreviated as poly-VPA) and a segment of polyethylene glycol) (sometimes abbreviated as PEG) slowly releases VPA in the living bodies of animals including humans and exerts the action and effect inherent in VPA.

Accordingly, main characteristics or aspects of the technical matters disclosed herein can include the following aspects:
Aspect 1: A block copolymer containing a polymeric valproic acid segment represented by Formula I: 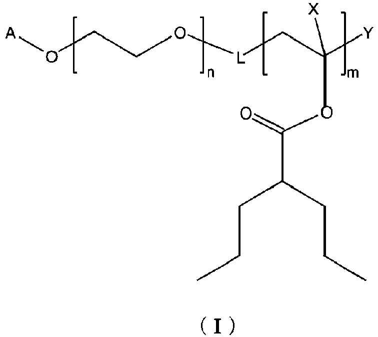 wherein,
   A represents unsubstituted or substituted C₁-C₁₂ alkyl, and a substituent when substituted represents a formyl group or a group of a formula R¹R²CH-, wherein R¹ and R² independently represent C₁-C₄ alkoxy, or R¹ and R² together represent -OCH₂CH₂O-, - O(CH₂)₃O- or -O(CH₂)₄O-;
   L represents a linking group;
   X represents H or C₁-C₆ alkyl, or H or C₁-C₃ alkyl, or H or methyl;
   Y represents H, SH, or S(C=S)-Ph or S(C=S)-OC₁-C₆ alkyl;
   m represents an integer of from 3 to 100, or from 5 to 60, or from 5 to 40, or from 8 to 40; and
   n represents an integer of from 5 to 10000, or from 10 to 1000, or from 20 to 500.
Aspect 2: The block copolymer acid of Aspect 1, wherein L is selected from the group consisting of groups represented by formulas:

   -(CH₂)_{b}-,

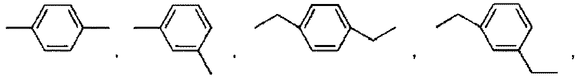

   and -(CH₂)_{b}S-, where each b is independently an integer of from 1 to 5. Here, when the linking group L is represented by-(CH₂)_{b}S-, the group is linked along the direction of description of Formula I. Specifically, a carbon atom of the terminal methylene of the group is bonded to the terminal oxygen atom (O) of PEG chains, which are m number of repeating units in Formula I, and the other terminal S atom is bonded to the α-terminal carbon atom of VPA-containing polymer chains, which are n number of repeating units in Formula I.
Aspect 3: A nanoparticle comprising the block copolymer of Aspect 1 or 2.
Aspect 4: A pharmaceutical preparation comprising the block copolymer of Aspect 1 or 2 as an active ingredient and physiological saline or any other additive or diluent commonly used in the art, wherein the pharmaceutical preparation is, for example, for use as an antiepileptic drug, a therapeutic drug for the manic state of bipolar disorders, a drug for suppressing the onset of migraine attack, a drug for suppressing diseases caused by abnormal activity of histone deacetylase (HDAC), or an antitumor agent.

### Advantageous Effects of Invention

The block copolymer comprising the polymerized valproic acid segment represented by the above Formula I, itself, slowly releases free valproic acid in the living bodies of animals including human. Also, without being bound by theory, the block copolymer may exist, in an aqueous medium (such as, but not limited to, buffered physiological saline according to need), as a nano-sized particle which comprises a core predominantly comprising a poly-VPA segment and a shell predominantly comprising a PEG segment. Such a particle may suppress the attachment or approach of, for example, proteins in the living bodies (including various enzymes) to the poly-VPA segment due to the presence of PEG chains that are highly mobile in an aqueous medium, and may suppress or modulate the release of free VPA from the particle or the action of any other active substance in the living bodies. Thus, the block copolymer provides the action and effect of VPA itself, and may exert excellent action and effect as a pharmaceutical product different from VPA, and can modify or enhance the chemical or biological properties of VPA. Therefore, it can contribute to the enrichment of VPA technologies in the field of medicine or pharmacy, without limitation.

### Brief Description of Drawings

FIG. 1 is ¹H NMR spectrum of the target substance (PEG-b-polyVPA) obtained in Production Example 4.
FIG. 2 is a particle size distribution diagram indicating results of dynamic scattered light measurement of the nanoparticle (Nano^{VPA}) obtained in Production Example 5.
FIG. 3 is a graphic representation of scoring of behavior patterns of mice by administration of the test drug in Test Example 1.
FIG. 4 is a graphic representation summarizing an effect of test drug administration on an expression level of brain-derived neurotrophic factor (BDNF) in Test Example 2.
FIG. 5 is a graphic representation concerning a therapeutic effect (survival rate) of test drug administration on C26 tumor-bearing cachexia model mice in Test Example 3.
FIG. 6 is a graphic representation indicating changes in body weight of model mice caused by test drug administration in Test Example 4.
FIG. 7 is a graphic representation indicating in vivo kinetics of VPA and Nano^{VPA} in mice in Test Example 5.

### Detailed Description of Invention

The technical matters disclosed herein, the terms described in relation to the present invention, or the like are used as having the meanings or contents that have been commonly used in the art unless otherwise mentioned. Regarding the technical matters, the following description may be further added.

The linking group represented by L of the block copolymer represented by Formula I generally means a group containing up to 34, or up to 18, or up to 10 carbon atoms and optionally oxygen and nitrogen atoms. Specific examples of the linking group can include those described in Aspect 2. With regard to the technical matters or the present invention, for example, C₁-C₁₂ alkyl means unbranched or branched alkyl having from 1 to 12 carbon atoms, such as, but not limited to, methyl, ethyl, propyl, isopropyl, n-butyl, isobutyl, tert-butyl, hexyl, octyl, decyl, dodecyl or the like; for example, C₁-C₆ alkyl or C₁-C₃ alkyl represents alkyl having 6 or 3 or less carbon atoms among the alkyls described above; C₁-C₆ alkyl in OC₁-C₆ alkyl is also the same as described above; and C₁-C₄ alkoxy has the same meaning as OC₁-C₄ alkyl.

The block copolymer can exist, in an aqueous medium (such as water, or buffered physiological saline according to need), as a nano-sized particle which comprises a core predominantly comprising a poly-VPA segments and a shell predominantly comprising PEG segments. The nano-sized particle is not limited, and may be in a range from 10 nm to 500 nm, or from 20 nm to 180 nm, or from 40 nm to 150 nm, as a hydrodynamic average diameter, as measured by a dynamic light scattering method, in an aqueous solution of the particle.

The block copolymer can be conveniently produced by protecting any terminal OH of PEG with a protecting group known in the art if necessary, modifying or altering the other terminal OH to a group or moiety known in the art (e.g. including terminal SH or S(=S) alkyl) capable of extending the repeating unit of alkyl vinyl valproate (preparation of a macromer initiator), and carrying out an extension (polymerization) reaction known per se in the art with alkyl vinyl valproate at the terminal.

The nano-sized particle from the block copolymer can be provided by dissolving the copolymer in a water-miscible organic solvent capable of dissolving the copolymer, and dialyzing the resulting solution against water through a dialysis membrane, for example, a dialysis membrane having cut-off pores of from 1 kDa to 50 kDa.

The nanoparticle (Nano^{VPA}) thus obtained can be separated from a solution or dispersion thereof by, for example, freeze-drying or centrifugation, and can thus be redissolved or suspended in physiological saline, according to need, and provide a preparation for oral or parenteral administration. In the case of a preparation for oral administration, the preparation can be provided in the form of a tablet or granule for oral administration, if necessary, using an additive, a diluent or the like which is commonly used in the art. Examples of the additive or diluent for oral preparations include, but are not limited to, Mg metasilicate aluminate, hydroxypropylcellulose, Mg stearate, ethylcellulose, hypromellose, glycerin fatty acid ester, talc, white sugar, precipitated Ca carbonate, polyoxyethylene (105) polyoxypropylene (5) glycol, powdered acacia, and titanium oxide as described above, and further chlorocarmellose sodium and sodium lauryl sulfate, which are used in controlled-release preparations of sodium valproate. In addition, the nanoparticle can be solubilized or dispersed in water, and thus can also be made into parenteral preparations, for example, solubilized or dispersed in physiological saline, and, if necessary, buffered.

The optimum dose of a composition comprising such a block copolymer or nanoparticle as an active ingredient can vary depending on the severity of a disease to be treated and the age, sex and body weight of a patient to be administered, and therefore cannot be uniquely specified. However, it can be determined by a specialist with reference to the usage, dose and the like of VPA sodium salt actually used clinically, if necessary, based on effective data obtained through small-scale clinical trials and the like.

Hereinafter, the disclosed matters or the present invention will be more specifically described based on the disclosed matters in the present specification or typical examples of the present invention for the purpose of avoiding complicated description, but the disclosed matters or the present invention are/is not limited to these examples.

### Production Example 1: Synthesis of vinyl valproate

5.6 mg of palladium (II) acetate (FUJIFILM Wako Pure Chemical Corporation) and 17 mg of potassium hydroxide (FUJIFILM Wako Pure Chemical Corporation) were added to 25 mL of vinyl acetate (FUJIFILM Wako Pure Chemical Corporation) and 17 mL of valproic acid (Tokyo Chemical Industry Co., Ltd.), and they were reacted at room temperature for 3 days. After acetic acid as a by-product was removed by an evaporator, 25 ml of vinyl acetate was added again, and they were reacted for 3 days. Palladium was removed by Celite filtration, and then vinyl valproate (20 mL) was obtained by distillation under reduced pressure.

### Production Example 2: Synthesis of CH₃O-(CH₂CH₂O)ₙ-CH₂PhCH₂Cl (T59)

To commercially available CH₃O-(CH₂CH₂O)ₙ-H (MW = 5000, 50 g, 10 mmol), tetrahydrofuran (THF) (100 mL) and 7.2 mL of butyllithium (11.5 mmol, 1.6 M-hexane) were added. Then, α,α'-dichloroparaxylene (17.5 g, 0.1 mmol) was added, and they were reacted at 50°C for 2 days. After precipitation in 2-propanol (IPA), the precipitate was dissolved in methanol for precipitation again in IPA. This operation was repeated three times, and the resulting pale yellow precipitate was dried under reduced pressure to give a target substance (47.2 g).

### Production Example 3: Synthesis of CH₃O-(CH₂CH₂O)ₙ-CH₂PhCH₂S(=S)OCH₂CH₃ (T64)

CH₃O-(CH₂CH₂O)ₙ-CH₂PhCH₂Cl (T59, 30 g) obtained in Production Example 1 was dissolved in 150 mL of methanol, potassium ethylxanthate (CH₃CH₂OC(=S)SK, 0.96 g) was added, and they were reacted at room temperature for 10 minutes. After centrifugal elimination of the precipitate, methanol was dried under reduced pressure. The residue was dissolved in chloroform and washed with water, and the chloroform layer was fractionated. After anhydrous sodium sulfate dehydration and filtration, the product was precipitated in IPA. After centrifugation, the product was dried under reduced pressure to give a target substance (27.4 g).

### Production Example 4: Synthesis of CH₃O-(CH₂CH₂O)ₙ-CH₂PhCH₂(CH₂CH(OC(=O)CH(CH₂CH₂CH₃)₂))ₘS(=S)OCH₂CH₃ (T71)

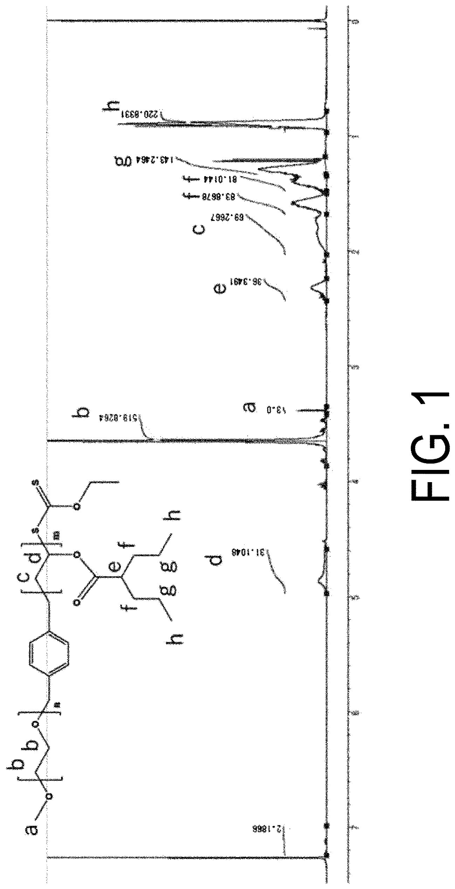

CH₃O-(CH₂CH₂O)ₙ-CH₂PhCH₂S(=S)OCH₂CH₃ (T64, 5.3 g) synthesized in Production Example 2, azobisisobutyronitrile (57 mg) and vinyl valproate (8.6 g) were added to a flask, subjected to nitrogen bubbling for 5 minutes, and then reacted at 60°C for 2 days. The resulting target substance was dissolved in THF, precipitated in IPA, and dried under reduced pressure to give a target substance (5.4 g). A ¹H NMR spectrum of the target product is presented in FIG. 1. From the proton peak values presented in FIG. 1 (see data in the table below), a repeat number m of vinyl valproate is derived as 36.0.

| | c | d | e | f | g | h |
|---|---|---|---|---|---|---|
| | | | | | | |
| proton | 2m | m | m | 4m | 4m | 6m |
| obs. | 69.3 | 31.1 | 36.3 | 164.9 | 143.2 | 221 |
| calc. | 34.65 | 31.1 | 36.3 | 41.2 | 35.8 | 36.8 |

### Production Example 5: Preparation of self-assembled particles

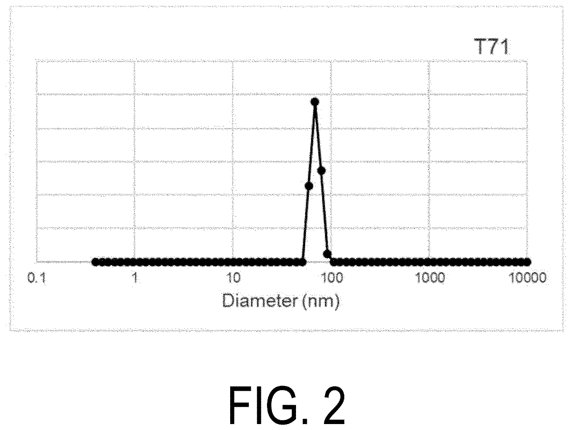

The copolymer synthesized above (T71) (4.46 g) was collected and dissolved in 44.6 mL of dimethylformamide (DMF), and the solution was put in a dialysis membrane (MWCO = 3.5 KDa) and dialyzed against 2 L of water. After the dialysis water was exchanged six times every 8 hours, dynamic light scattering measurement was performed, and it was confirmed that particles with an average diameter of 69 nm were formed. The results of the dynamic light scattering measurement are presented in FIG. 2.

### Test Example 1: Antiepileptic effect using pentylenetetrazole-induced epilepsy kindling model mice

Pentylenetetrazole (PTZ) kindling model mice were prepared by intraperitoneal administration (30 mg/kg) of PTZ (Day 1, 3, 5, 8, 10, 12). The dosage was increased to 33 mg/kg (Day 15) and then increased by 2%.

Valproic acid or the valproic acid particles (Nano^{VPA}) prepared in Production Example 5 was/were intraperitoneally administered (100 mg/kg (converted in terms of VPA amount)), and the effect was analyzed by behavior observation. VPA or Nano^{VPA} was administered on Days 0, 2, 4, 7, 9, 11, 14 and 21.

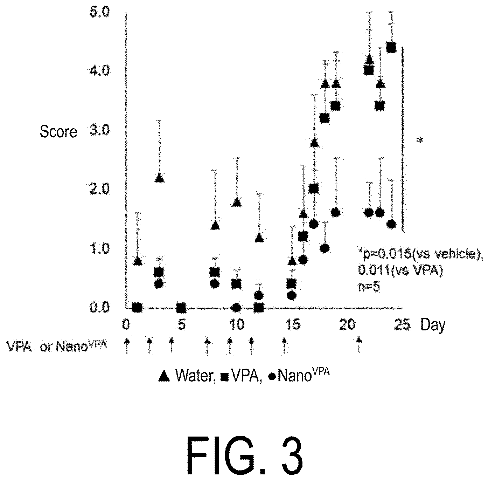

Scoring of behavior patterns of the mice confirmed that convulsions were suppressed by Nano^{VPA} administration. The results are presented in FIG. 3.

The scoring is based on the following scoring criteria:
Score 1: akinesia, exploratory behavior, grooming; Score 2: nodding behavior, short myoclonus; Score 3: long myoclonus, repetitive head movement, repetitive shaking behavior, tail stiffness; Score 4: clonic convulsion, forelimb rotational seizures, hindlimb abduction, persistent rearing behavior, falling, straub tail, kangaroo-like posture; Score 5: tonic convulsion, tonic clonic convulsion in which righting posture cannot be maintained.

### Test Example 2: Quantification of brain-derived neurotrophic factor (BDNF)

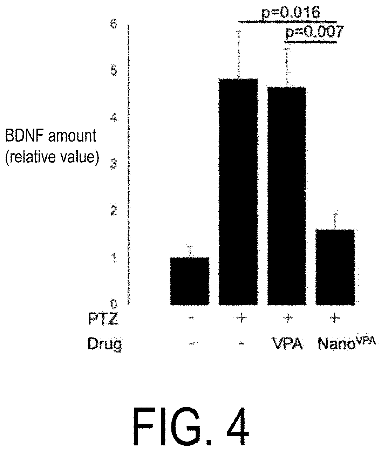

BDNF has been reported to be elevated in PTZ-induced epilepsy kindling model mice. As to the effect of drug administration on the expression level of BDNF, the hippocampus of the mice derived from Test Example 1 was collected and analyzed by RT-PCR. The results are presented in FIG. 4. From this figure, it can be confirmed that the expression level of BDNF was increased by PTZ, but that the expression level was decreased by Nano^{VPA} administration.

### Test Example 3

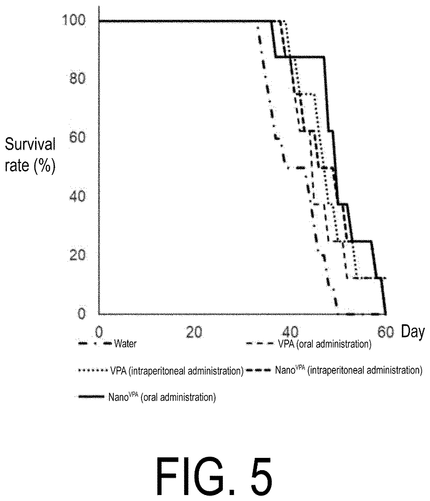

Valproic acid has histone deacetylase inhibitory activity, and thus has been reported to have a therapeutic effect in C26 tumor-bearing cachexia model mice. As to whether the same effect could be confirmed by Nano^{VPA} administration, the effect was analyzed using cancer cachexia model mice. C26 cells used in the tumor cachexia models were subcutaneously transplanted into Balb/c mice, and tumor-bearing mice were prepared. VPA or Nano^{VPA} was orally or intraperitoneally administered to the tumor-bearing mice, and the survival rate after administration was observed. The results are presented in FIG. 5. From this figure, improvement in survival rate is seen in the Nano^{VPA}-administered group.

### Test Example 4: Toxicity evaluation

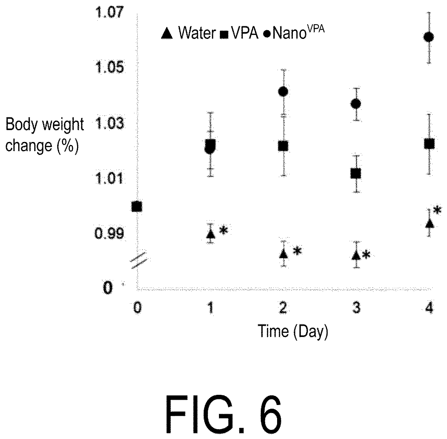

To evaluate the toxicity of VPA and Nano^{VPA}, VPA and Nano^{VPA} were intraperitoneally administered at a dose of 500 mg/kg for 5 days, and changes in body weight of the mice were observed. The results are presented in FIG. 6. As can be seen from this figure, the VPA-administered group indicated a decrease in body weight, but the Nano^{VPA}-administered group indicated no decrease in body weight.

### Test Example 5: In vivo kinetics of VPA and Nano^{VPA}

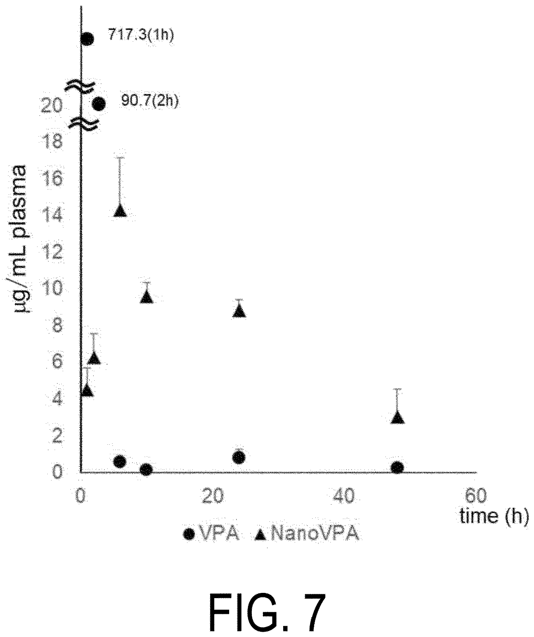

Six (6)-week-old ICR mice (male) were intraperitoneally administered with sodium valproate (VPA) or valproic acid nanoparticles (Nano^{VPA}) at a valproic acid concentration of 400 mg/kg. After lapse of a predetermined time, the mice were dissected, and the blood was collected in a heparinized tube and centrifuged to give plasma. The valproic acid concentration in plasma was analyzed by Nanopia TDM valproic acid (Sekisui Medical Co., Ltd.). The results are presented in FIG. 7. As can be seen from the figure, the formation of nanoparticles of VPA makes it possible to retain valproic acid as an active ingredient in blood for a long period of time and to maintain its effect.

### Industrial Applicability

The alterations or modifications of valproic acid according to the invention can be applied in the medical and pharmaceutical industries, for example for allowing valproic acid to exert its own action and effect, without the use of specific combinations of various carriers or additives.

## Claims

1. A block copolymer comprising a polymerized valproic acid segment represented by Formula I: 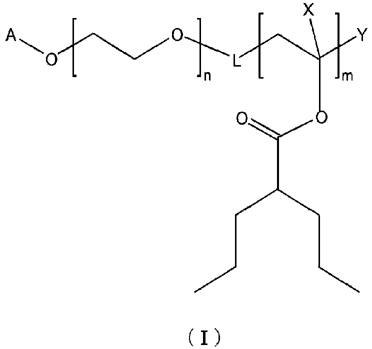 wherein,
A represents unsubstituted or substituted C₁-C₁₂ alkyl, and a substituent when substituted represents a formyl group or a group of a formula R¹R²CH-, wherein R¹ and R² independently represent C₁-C₄ alkoxy, or R¹ and R² together represent - OCH₂CH₂O-, -O(CH₂)₃O- or -O(CH₂)₄O-;
L represents a linking group;
X represents H or C₁-C₆ alkyl;
Y represents H, SH, or S(C=S)-Ph or S(C=S)-OC₁-C₆ alkyl;
m represents an integer of from 3 to 100; and
n represents an integer of from 5 to 10000.

2. The block copolymer according to claim 1, wherein L is selected from the group consisting of groups represented by formulas:
-(CH₂)_{b}-, 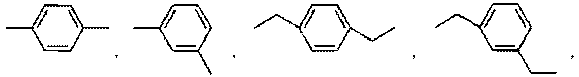
and -(CH₂)_{b}S-, wherein each b is independently an integer of from 1 to 5.

3. A nanoparticle comprising the block copolymer according to claim 1.

4. A pharmaceutical preparation comprising a block copolymer according to claim 1 as an active ingredient and physiological saline or an additive or diluent commonly used in the art.

5. The pharmaceutical preparation according to claim 4, which is for use as a medicament selected from an antiepileptic drug, a therapeutic drug for a manic state of bipolar disorders, a drug for suppressing an onset of migraine attack, a drug for suppressing diseases caused by abnormal activity of histone deacetylase (HDAC), and an antitumor agent.
